# EUROPEAN PATENT SPECIFICATION

(11) **EP 1 143 986 B1**
(45) Date of publication and mention of the grant of the patent: **02.08.2006**
(21) Application number: 99960165.1
(22) Date of filing: 26.10.1999
(51) Int. Cl.: A61K 36/00, A61K 36/28, A61K 36/906, C07C 47/225, C07D 311/32, A61P 31/10, A61P 33/02, C07D 303/40

(54) **PLANT-DERIVED ANTI-PARASITIC AND ANTIFUNGAL COMPOUNDS AND METHODS OF EXTRACTING THE COMPOUNDS**
PFLANZLICHEN ANTI-PARASITÄREN UND ANTIFUNGALEN VERBINDUNGEN UND VERFAHREN ZUR EXTRAKTION VON DIESEN VERBINDUNGEN
COMPOSES ANTIPARASITAIRES ET ANTIFONGIQUES DERIVES DE PLANTES ET PROCEDES D'EXTRACTION DE CES COMPOSES

(30) Priority: 27.10.1998 US 105888 P
(43) Date of publication of application: 17.10.2001
(73) Proprietor: Walter Reed Army Institute of Research, Washington, DC 20307 (US)
(72) Inventor: OKUNJI, Christopher, O., Silver Spring, MD 20906 (US); IWU, Maurice, M., Silver Spring, MD 20906 (US); JACKSON, Joan, E., Rockville, MD 20853 (US); TALLY, John, D., Jr., Washington, DC 20018 (US); BACCHI, Cyrus, East North Port, NY 11731 (US); AYAFOR, Johnson, F., Dschang (CM)
(74) Representative: Weyland, J.J. Pierre
(86) International application number: PCT/US1999/025344
(87) International publication number: WO 2000/024411

(56) References cited:
- SAMUEL F. KIMBU ET AL.: "A NEW LABDANE DITERPENOID FROM THE SEEDS OF AFRAMOMUM DANIELLII." JOURNAL OF NATURAL PRODUCTS., vol. 50, no. 2, March 1987 (1987-03) - April 1987 (1987-04), pages 230-231, XP000891749 XX, XX ISSN: 0163-3864
- J. FOYERE AYAFOR ET AL.: "NOVEL BIOACTIVE DITERPENOIDS FROM AFRAMOMUM AULACOCARPOS." JOURNAL OF NATURAL PRODUCTS., vol. 57, no. 7, July 1994 (1994-07), pages 917-923, XP000891752 XX, XX ISSN: 0163-3864

## Description

This application claims priority to U.S. provisional patent application serial No. 60/105,888, filed on October 27, 1998, the complete disclosure of which is incorporated herein by reference.

Part of this work was completed while holding a National Research Council Associateship at the Walter Reed Army Institute of Research.

### 1. FIELD OF THE INVENTION

The present invention relates to the use of biologically active extracts from *Aframomum aulocacarpus, Eupatorium odoratum, Glossocalyx brevipes and Napoleonaea imperialis,* for the manufacture of a medicament for use in the treatment of determined protozoa diseases.

### 2. BACKGROUND OF INVENTION

Available drugs for the treatment of diseases due to various protozoal infections are inadequate due to increasing parasite resistance and serious toxicity associated with some of them. There is therefore a need for new and effective therapeutic agents. In general, antiprotozoals are not given high priority for commercial development because the per capita health expenditure in many tropical countries is less than the cost of one course of drug therapy. Thus, many "modern" antiparasitic drugs were initially marketed more than 40 years ago.

Clinical intervention in the treatment of limited of leishmaniasis, for example is limited to the use of pentavalent antimonials (SbV), sodium stilbogluconate and N-methylglucamine antimonate, and secondarily, amphotericin or pentamidine. Croft, S.L., 1988, Recent developments in the chemotherapy of leishmaniasis, *Trends Pharmacol. Sci.* 9. 376., Bryceson, A., 1987, Therapy in man. In *The Leishmaniases in Biology and Medicine,* Vol. 2, *Clinical Aspects and Control, W.* Peters, and R. Killick-Kendrick, eds., Academic Press, New York, 847. Treatment with these agents is not consistently effective particularly for the most virulent leishmanial disease forms (Jha, T.K., 1983, Evaluation of diamidine compound (pentamidine isethionate) in the treatment of resistant cases of kala-azar occurring in North Bihar, India, *Trans. Roy. Soc. Trop. Med. Hyg.* 77:167., Rocha, R.A.A., Sampaio, R.N., Guerra, M., Magalhaes, A., Cuba, C.C., Barreto, A.C., and Marsden, P.D., 1980, Apparent Glucantime failure in five patients with mucocutaneous leishmaniasis, *J. Trop. Med. Hyg.* 83:131-139. Mebrahtu, Y;B., Lawyer, P., Githure, J., Were, J.B., Muigai, R., Hendricks, L., Leeuwenburg, J., Koech, D., Roberts, C., 1989, Visceral leishmaniasis unresponsive to pentostam caused by *Leishmania tropica* in Kenya, *Am. J*. *Trop. Med. Hyg.* 41:289.

The four drugs most frequently used to treat leishmaniasis all require parenteral administration, use dates back to 40 to more than 50 years, and all have such severe side-effects that treatment only in a hospital setting is recommended (Bryceson, 1968,1987). No antileishmanial is Food and Drug Administration (FDA) approved, and Pentostam® (sodium antimony gluconate) can be administered only via "investigational protocol" in the USA. There is no chemoprophylaxis for any leishmanial disease. An aminoquinoline, WR6026, having showed initial promising results in animal models for visceral leishmaniasis, has not proven effective in human clinical testing either in Africa or South America (Division of Experimental Therapeutics, Walter Reed Army Institute of Research, unpublished observations). Liposomal Amphotericin B has not proven efficacious against *Leishmania* species known to cause mucocutaneous disease (Wortmann, et al., 1997). Topical treatment for leishmanial disease is not effective even for cutaneous disease forms because leishmaniasis is a systemic disease (Neva, et al., 1997). There is no general vaccine for leishmaniases, although a live vaccine is used in the Middle East for certain *Leishmania (Leishmania) tropica*/*Leishmania (Leishmania) major* to prevent facial scarring.

Drug resistance is so severe in certain endemic regions that thousands are dying in India of untreatable, multi-drug resistant visceral leishmaniasis; and in Northern Africa as a result of malnutrition exacerbated disease (Anonymous, 1993; Cerf, et al., 1987; de Beer, et al., 1991; Sundar, 1997).

Immunodeficiency, either as the result of leishmanial tubercular- or HIV co-infections, poses serious therapeutic difficulties as leishmanial coinfection is reported to potentiate the pathology of both these bacterial and viral infections (Alvar, et al., 1997; Bernier R, et al., 1995; Bryceson, 1987; Faraut-Gamarelli, et. al., 1997). Terra and coworkers (1996) proposed to include visceral leishmaniasis as an opportunistic infection in the IVC-2 group of clinical classification of HIV infection; and Montalban, et al., (1990b), in the CDC group IVC-1 as indicative of AIDS (also, Federico, et al., 1989; Montalban, et al., 1990).

Global travel and commerce result in patients having complex disease exposure history, and transportation of leishmanial parasites far from their anticipated endemic regions making both diagnosis and patient management difficult (Albrecht, et al., 1996). Leishmaniases have an annual incidence of 2-3 million new cases per year with 12 million infected and 350 million at risk in 88 countries worldwide (Anonymous, 1990; Croft, 1988; World Report on Tropical Diseases, 1990). However, these figures do not take into consideration that leishmanial disease transmission may result in asymptomatic infections which when the immunologic cell mediated response is depressed due for example to co-infection (HIV), natural ageing, pregnancy, immunodepressant drugs, malnutritian, etc. The disease symptoms can recur, sometimes more than 30 years post-infection.

Two major groups of diseases caused by flagellate protozoa are African sleeping sickness *(Trypanosoma brucei* spp.) and trichomoniasis *(Trichomonas* /*Tritrichomonas).* African trypanosomiasis affects both domestic and wild animals as well as humans in mainly rural settings (Kuzoe, 1993; WHO, 1995) while trichomoniasis is a cosmopolitan disease in men as well as women, and a threat to cattle breeding in most agricultural areas of the world (Hammill, 1989; Levine, 1985). Treatment of the organisms causing these diseases presents problems, in part, due to lack of new, inexpensive agents, the toxicity of existing agents, and the development of resistance to existing drugs (Kuzoe, 1993; Lossick, 1989).

African trypanosomiasis is endemic in over 10 million square kilometers of sub-Saharan Africa, affecting humans and all domesticated livestock (WHO, 1995). There are an estimated 25,000 new cases of human disease yearly and an animal incidence of 250-300,000 cases but these estimates are low, based on recent civil unrest and lapses in local tsetse fly control and medical surveillance (WHO, 1995; F. Kuzoe, pers. commun.). The primary drugs for human and veterinary trypanosomiasis have been in use for >50 years. Resistance is spreading, especially to the diamidines, pentamidine and Berenil®, and melarsoprol (Arsobal®), the only available agent for late stage (CNS) human disease (van Nieuwenhove, 1992; Kuzoe, 1993). Melarsoprol is also toxic, with a 3-5% incidence of cerebral episodes reported (Pepin, & Milord 1994; Wery, 1994). A recent FDA-approved drug, difluoromethylornithine (DFMO. Eflornithine®), is effective but expensive for use in economically deprived areas ($450 / patient: WHO, 1995). Veterinary trypanocides include diminazene (Berenil®) and isometamidium (Samorin®) which are used prophylactically for control of disease in cattle herds (WHO, 1995; Kaminsky et al., 1993). Resistance to both agents has been documented in field studies (Kuzoe, 1993; Schoenfeld et al., 1987; Williamson, 1970). For these reasons, there is an urgent need to develop new trypanocides.

*Trichomonas vaginalis* is a sexually transmitted pathogen of the human urogenital tract. It infects the vaginal epithelium, causing severe irritation and the development of a discharge. Trichomoniasis is one of the most prevalent STD's in the Western world, accounting for a large number of visits to private gynecologists and public clinics (Hammill, 1989). In addition to social distress caused by the disease, recent evidence suggests a high incidence rate between cervical cancer and trichomoniasis (Gram et al., 1992). The disease is widespread, with about 3 million cases in women annually in the United States alone (Hammill, 1989).

Chemotherapy for human trichomoniasis relies on a group of 5'-nitroimidazoles, with metronidazole (Flagyl®) being the most utilized. In the United States, metronidazole is the only available agent, although other derivatives are used in Europe and other areas. Since metronidazole has been in continuous use since 1955, there has been increasing reports of metronidazole-resistant vaginitis (Meingassner & Thurner, 1979; Wong et al., 1990; Voolman & Boreham, 1993). Because of its potential to produce free radicals upon reduction, it is potentially mutagenic and not given to pregnant women (Lossick, 1989). At present, there is no alternative to the 5'-nitroimidazoles for therapy of metronidazole-refractory disease, nor for treatment of pregnant women.

*Trichomonas foetus* is the agent of bovine trichomoniasis, causing reproductive failure. Parasites are spread by infected bulls, multiply in the vagina and invade the cervix and uterus. One to 16 weeks after breeding, abortion of the fetus occurs (Levine, 1985). If the placenta and fetal membranes are eliminated following abortion, the cow may spontaneously recover. If some of these tissues remain inside the animals, permanent sterility may result. There is no satisfactory treatment for treatment of diseased cows, while treatment of bulls is tedious and expensive. Aminoquinuride (Surfen®) or acrifiavine (Trypaflavine®) may be used topically, with dimetridazole injected into the urethra. Unless the bull is valuable, it is usually destroyed (Levine, 1985).

The disease is common in open range breeding ranches and may reach epidemic levels. In Australia, 40-65% of cattle were reported to be infected, while the prevalence in California was reported to be 14% (Yule et al., 1989). The economic losses due to bovine trichomoniasis have been estimated to be $665 per infected dairy cow, while the widespread prevalence of the disease would account for tens of millions of dollars annually (Yule et al., 1989).The overall situation for chemotherapy of trichomoniasis therefore, is the reliance on a single drug as drug class for chemotherapy of human disease, and no effective control measures for bovine trichomoniasis.

Another protozoan disease, malaria, remains the greatest human killer among parasitic infections, despite the world-wide effort to combat the disease and attempts at the eradication of the causative organisms. The emergence of multi-drug resistant strains of *Plasmodium falciparum,* the most lethal of the malaria parasites, poses a serious health-care problem, not only in the malaria-endemic countries but also among international travellers.

Similarly, fungal and yeast infections are becoming increasingly resistant to modern drugs. In immunologically compromised individuals, for example, complications arising from uncontrollable fungal infections are among the leading cause of death. There is, therefore, a need for new and effective alternative treatment.

Protozoan infections are also a major cause of mortality and morbidity in immunosuppressed patients, as in acquired immunodeficiency syndrome (AIDS). A single therapeutic agent active against different types of protozoa would be a major innovation in the treatment of these diseases It would therefore be useful to develop more effective, less toxic and orally active antiprotozoal agents. There are reports on the potential of plants as sources of new antiprotozal agents. A series of investigations on the antiprotozoal activity of plant from West and central Africa had be conducted. For example the antileishmanial and antimicrobial activities of Nigerian medicinal plants, have been evaluated by Iwu (1992), Okunji, et. al. (1990, 1991, 1996, pg. 9(3)). For example, this method relies on drug inhibition of parasite production of ¹⁴CO₂ from a battery of ¹⁴C-substrates to detect drug-mediated parasite damage at low drug concentration within a short time (Jackson et al., 1989, 1990).

Thus, there is an urgent need for new biologically active compounds for use in treating protozoa diseases which avoid the harmful side-effects of conventional pharmaceuticals.

### SUMMARY OF THE INVENTION

An objective is to identify biologically active compounds for use in treating protozoa diseases which avoid the harmful side-effects of conventional pharmaceuticals.

The above objective is obtained by biologically active extract from at least one plant selected from the group consisting of *Aframomum aulocacarpus, Eupatorium odoratum, Glossocalyx brevipes* and *Napoleonaea imperialis.*

The present invention is based on the finding that an extract from *Eupatorium odoratum* exerts anti-leishmanial or anti-malarial effects; an extract from *Glossocalyx brevipes* exerts anti-trichomonal effect; an extract from *Aframomum aulacocarpus* exerts anti-malarial or anti-trypanosomal effects; and an extract from *Napoleonaea.imperialls* exertrs anti-leishmanial or anti-trypanosomal effect.

Accordingly, the present invention encompases the uses specified in the claims.

### BRIEF DESCRIPTION OF THE DRAWINGS

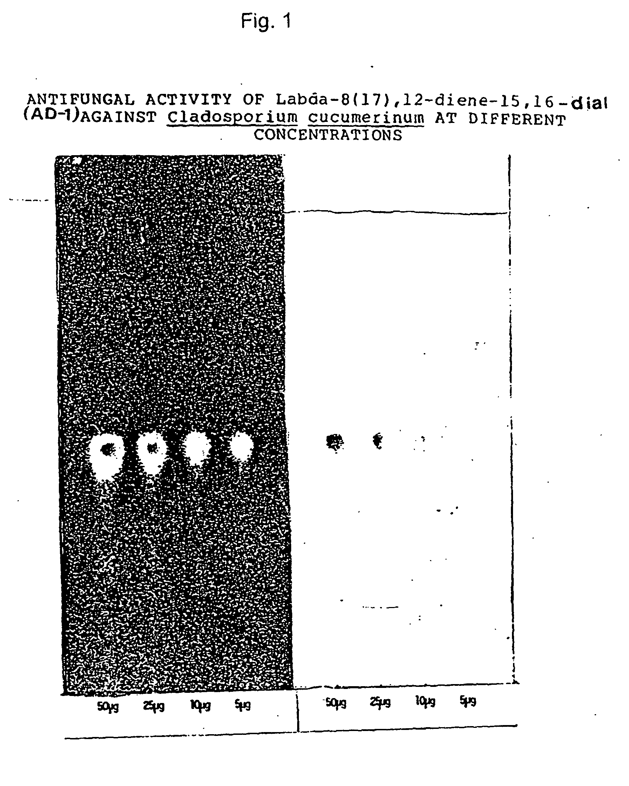

- Fig. 1: illustrates a thin layer chomatography (TLC)-bioassay on a silica gel plate, showing inhibition of the fungus, *Cladosporium* cucumerinum, by, Labda-8(17),12-diene-15,16-dial (reference example) at four concentration levels.

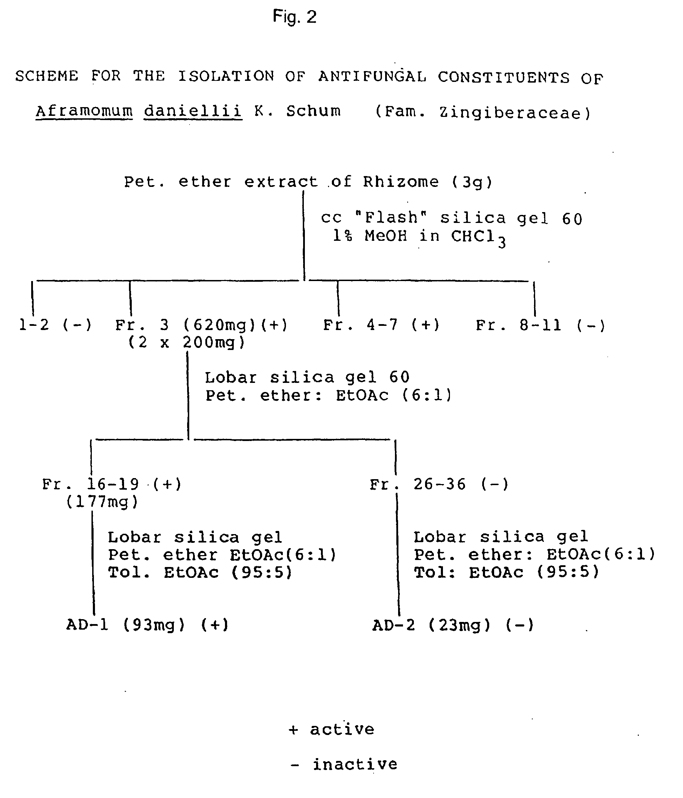

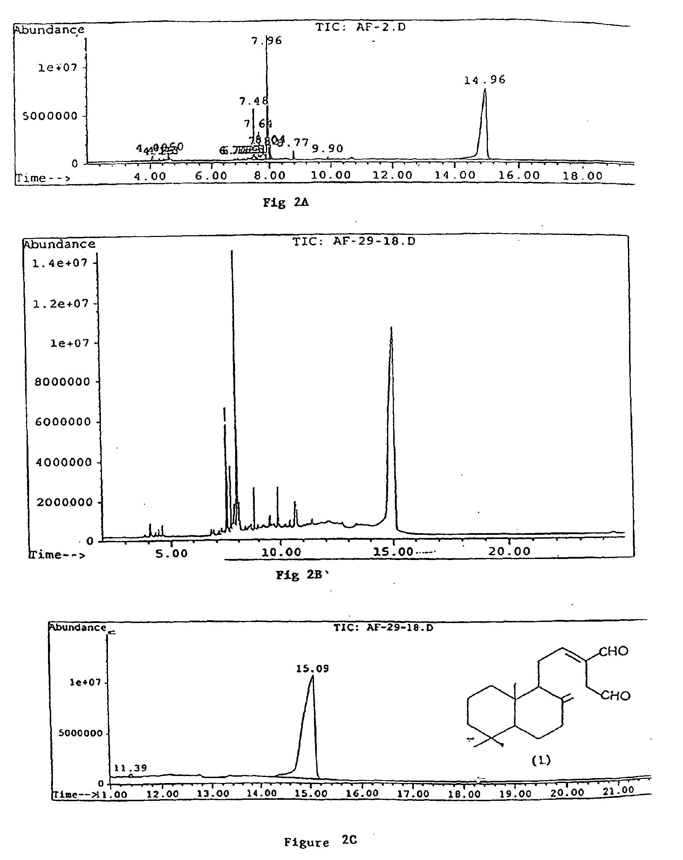

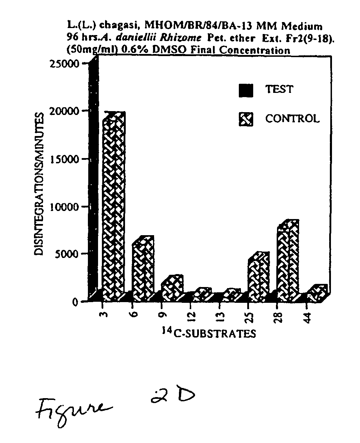

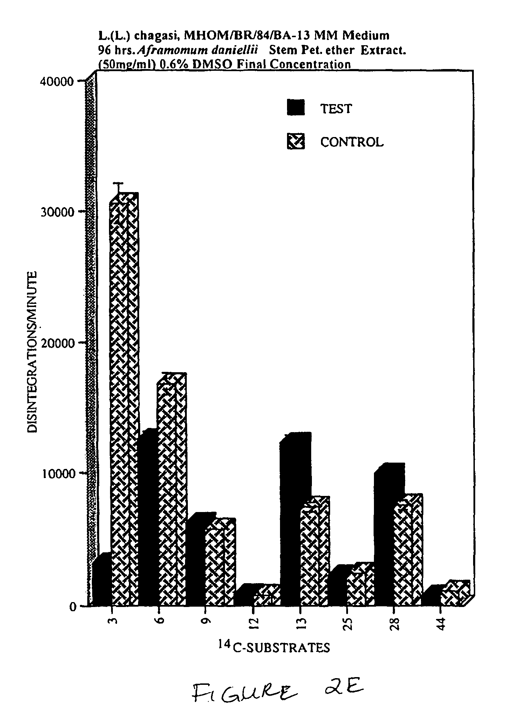

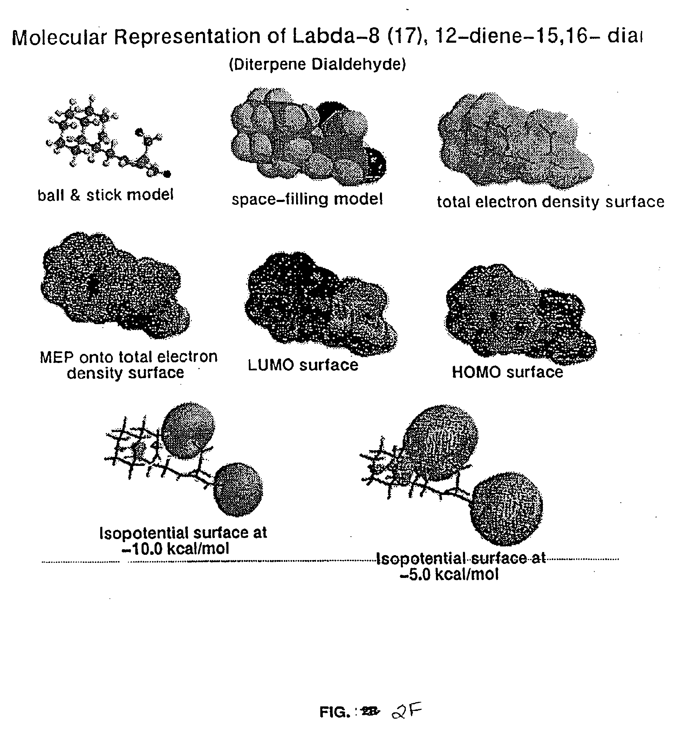

- Figs. 2, 2A-2F: illustrate radiorespirometric (RAM) data showing markedly reduced respiration of Leishmania (Leishmania) chagasi, a visceral disease parasite after, Labda-8(17),11-diene-15,16-dial (reference example) treatment in vitro. The vehicle-control-treated parasite respiration is represented by the light grey vertical bars; the Labda-8(17),12-diene-15,16-dial (50 ug/ml for 96 h) treated parasites, by the solid black bars. The ¹⁴C-substrate numeric codes (x-axis) are provided.

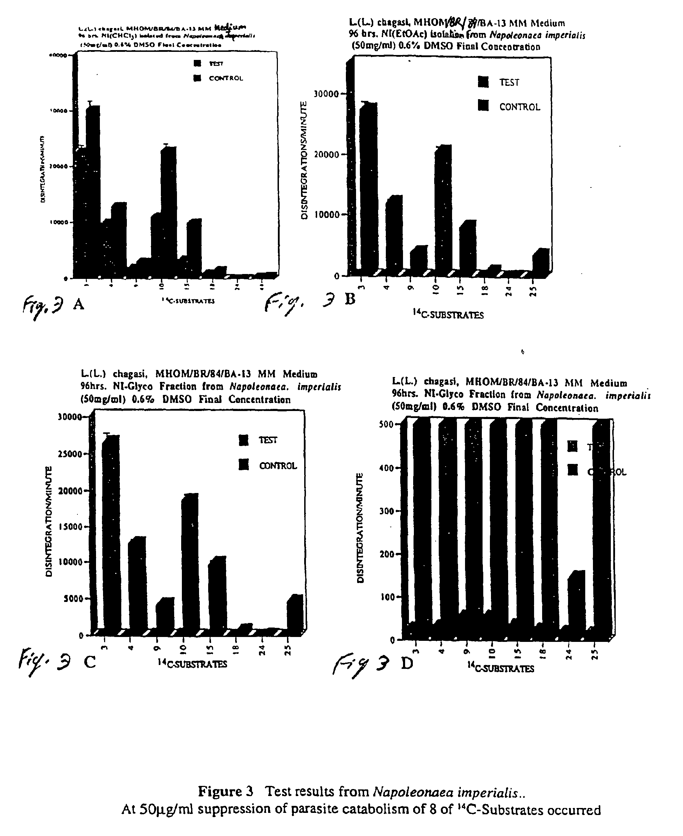

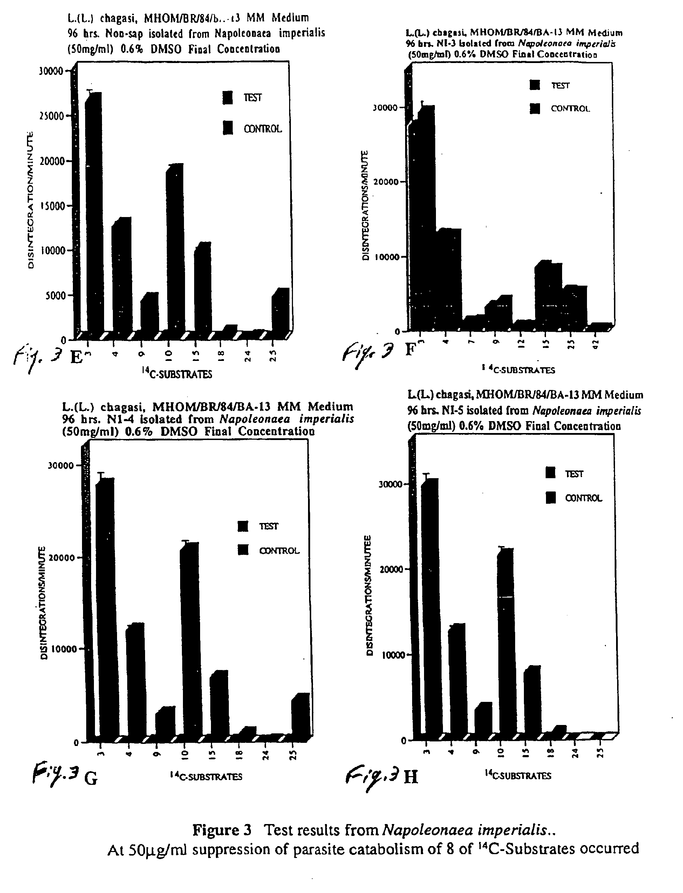

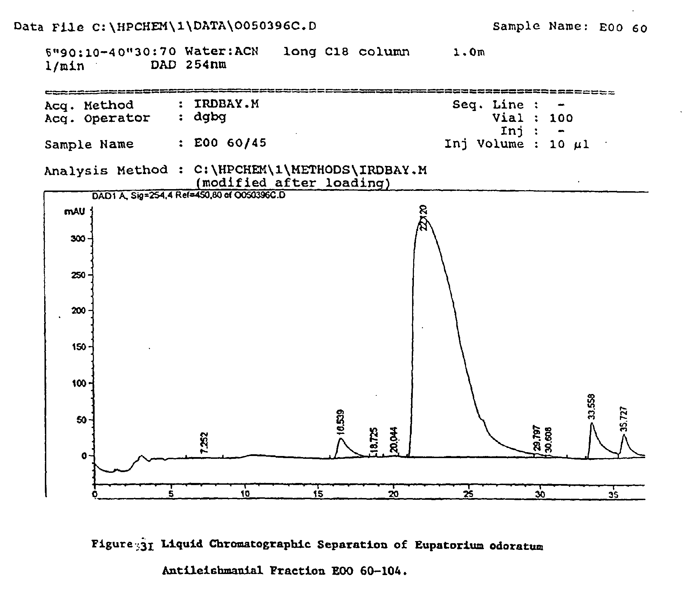

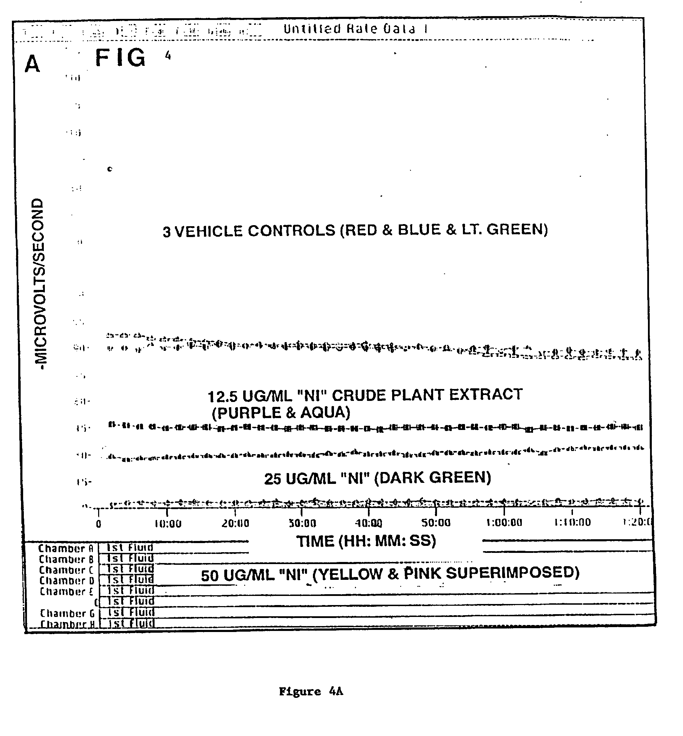

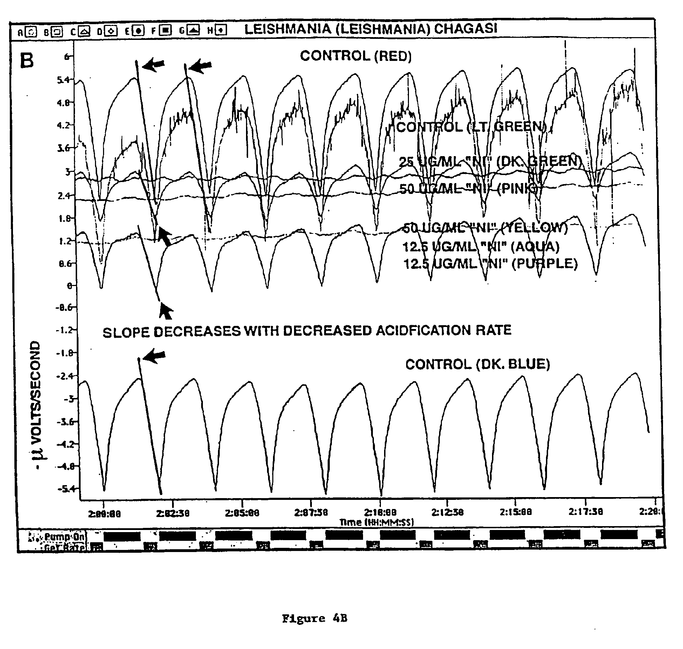

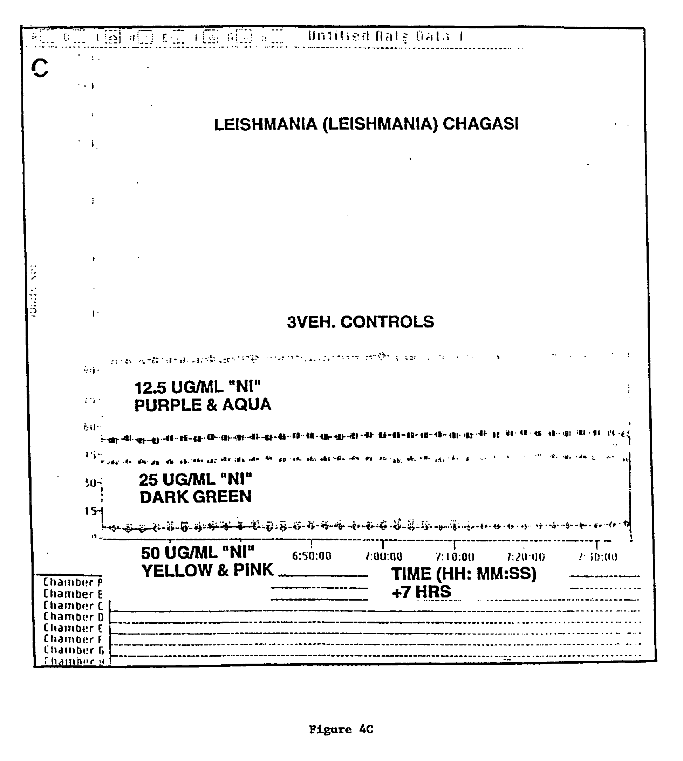

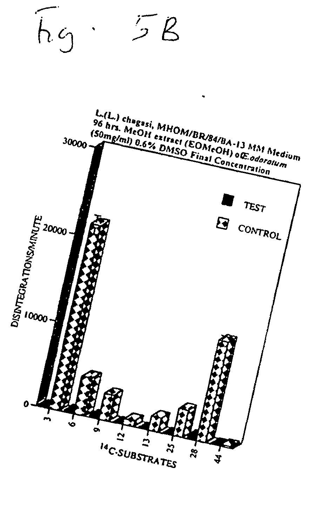

- Figs. 3A-3I: illustrate cytosensor microphysiometer (CMS) antileishmanial promastigote results after 17.5 h *Napoleonaea imperialis* treatment. The duplicate control parasite (i.e. parasites treated with drug solvent, 0.6% DMSO) tests, represented as uppermost lines, "G" and "H", have a consistently higher metabolic rate during the 11 h of observation. Parasites preincubated in parallel with controls for 17.5 h with 6.3- (lines "A" and "B"), 12.5- (lines "C" and "D"), and 50µg ml⁻¹ *Napoleonaea imperialis(lines* "E" and "F"), manifest lower metabolic rates, with the two highest drug concentrations resulting in metabolic rates very close to zero.
- Figs. 4A-4C: illustrate radiorespirometric (RAM) data showing markedly reduced respiration of *Leishmania (Leishmania) chagasi,* a visceral disease parasite after, Labda-8(17),12-diene-15,16-dial (reference example) treatment *in vitro.* The vehicle-control-treated parasite respiration is represented by the light grey vertical bars; the Labda-8(17),12-diene-15,16-dial (50µg ml⁻¹ for 96 h) treated parasites, by the solid black bars. The ¹⁴C-substrate numeric codes (x-axis) are provided.

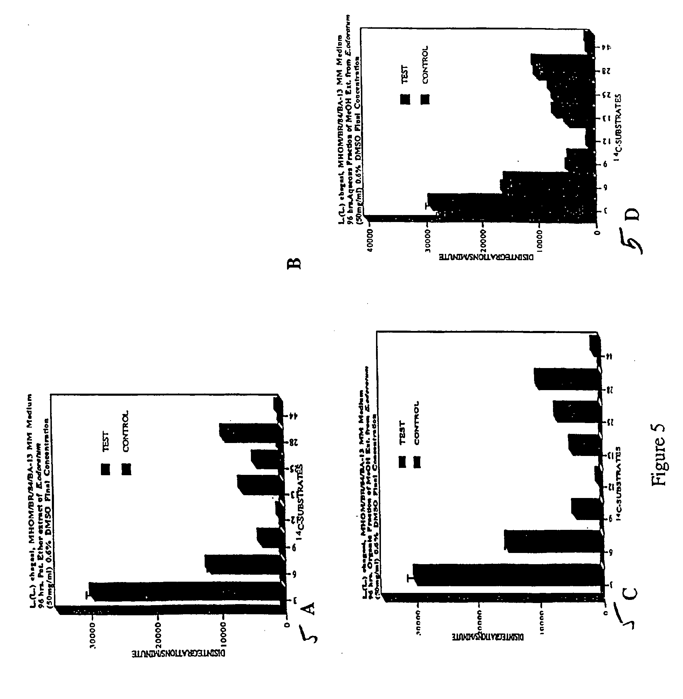

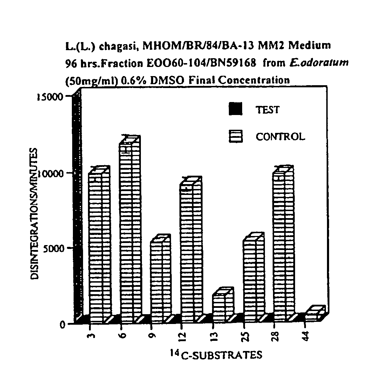

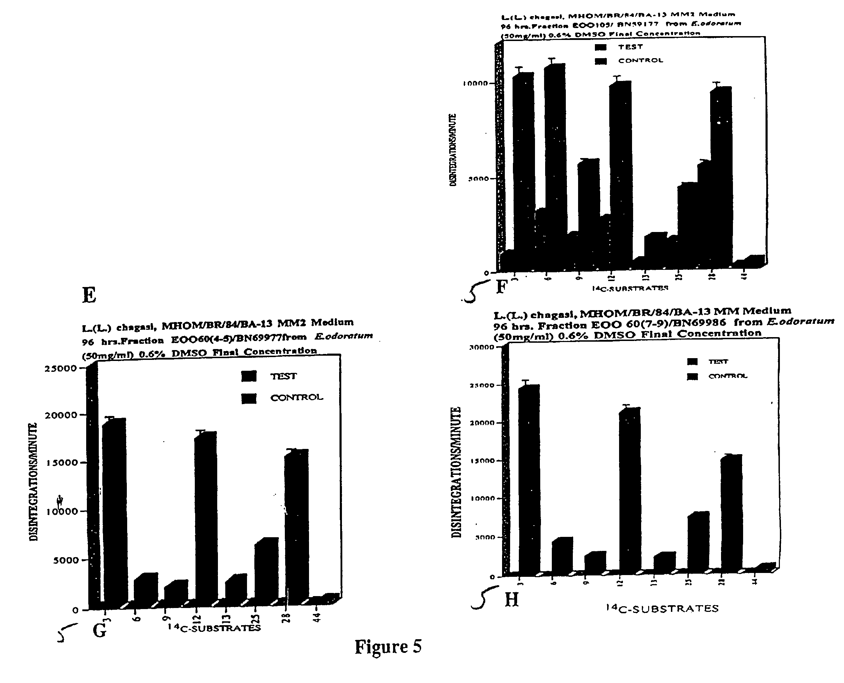

- Figs. 5A-5H: illustrate radiorespirometric (RAM) data showing markedly reduced respiration of *Leishmania (Leishmania) chagasi,* a visceral disease parasite after *Eupatorium odoratum* treatment *in vitro.* The vehicle-control-treated parasite respiration is represented by the light grey vertical bars; the E. odoratum extracts treated parasites, by the solid black bars. The ¹⁴C-substrate numeric codes (x-axis) are provided.

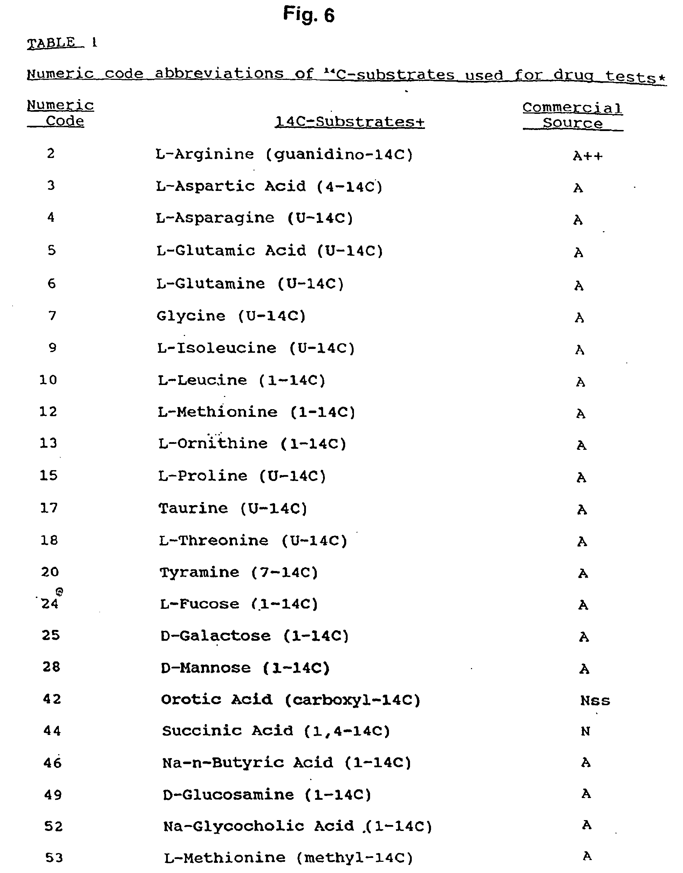

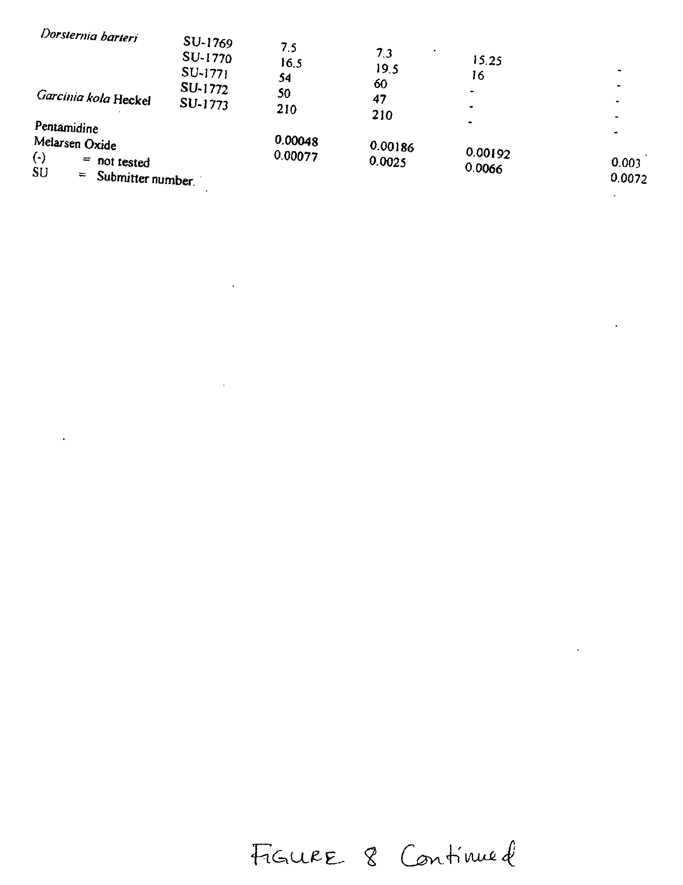

- Figs. 6-9: illustrate Tables 1-4.

### DETAILED DESCRIPTION OF PREFERRED EMBODIMENTS

The therapeutic potential of medicinal plants based on a battery of biologic test systems, including radiorespirometric, Cytosensor®, bioautography, agar dilution methods, and MALARIA test method & Trypanosomails test method, etc., are described. Bioassay-guided chromatographic fractionation of the active crude extracts led to the isolation, identification and structure elucidation of bioactive compounds. Exemplary sources of active ingredients include labdane-dial from *Aframomum daniellii* (reference example)*, aframodial* from *Aframomum aulacocarpus,* Mannispirostan A from *Dracaena* mannii (reference example), and Sakuranetin from *Eupatorium odoratum .* Other sources include *Glossocalyx brevipes* and *Napoleonaea imperialis.*

Compounds having antiprotozoal properties are identified using bioassay-directed fractionation. Previously, we reported the Evaluation of Plant Extracts for Antileishmanial Activity Using a Mechanism-Based Radiorespirometric Microtechnique (RAM). *Iwu, M.M., Jackson, J.E., Tally, J.D., and Klayman, D.L., Evaluation of Plant Extracts forAntileishmanial Activity Using a Mechanism-Based Radiorespirometric Microtechnique (RAM Planta Medica,* (1992) 58: *436-441.,* Recently the biological activity of saponins from two *Dracaena* species,was reported by our group Okunji C.O., Iwu M.M, Jackson J.E. and Tally J.D., Biological Activity of Saponins from Two *Dracaena* species, *Advances in Experimental Medicine & Biology,* 404:415-28,1996, antifungal and several molluscicidal constituents of D. *mannii* (Okunji *et al.,* 1990; 1991).

The present invention is based on the identification of an extract from *Eupatorium odoratum* as anti-leishmanial or anti-malarial agent; an extract from *Glossocalyx brevipes* as anti-trichomonal agent; an extract from of *Aframomum aulacocarpus* as anti-malarial or anti-trypanosomal agent; and an extract from *Napoleonada imperialis* as anti-leishmanial or anti-trypanosomal agent.

### MATERIALS AND METHODS

### Plant Materials

All plant materials utilized were collected near the Nsukka campus of the University of Nigeria with the exception of *Aframomum aulacocarpus* which was collected at Bamenda, Cameroon. The collection in Nigeria was chosen primarily from plants listed in an ethnomedicinal survey carried out among the lgbo people (lwu, 1981/82, 1993). The plants were taxonomically identified by Mr. A. Ozioko of the Department of Botany, University of Nigeria, Nsukka and the identities confirmed by Dr. J. C. Okafor of the Forestry Herbarium, Enugu. Voucher specimens have been deposited at the Bioresources Development and Conservation programme Herbarium at Nsukka, Nigeria. Prior to extraction, the plant materials were oven dried at 40°C and the dried vegetable drugs were ground to coarse powder.

For column chromatography (CC), Flash CC silica gel 60 size 0.063-0.200. mm (70-230 mesh ASTM, EM Science, was used, and Sephadex LH-20, Sigma, for gel filtration. Lichro-prep silica gel 60 (40-60um, Merck), Low-pressure liquid chromatography (Lobar) was done using a LichroPrep RP-8 column (40-63 mm 2.5 X 25 Merck) equipped with an FMI pump. DCCC equipment consisted of (Tokyo Rikakikai Nishikawa Bldg Toyama-Cho Kanda Chiyoda, Tokyo, type 300 glass tubes (length 400 mm, I.D. 2 mm), solvent system: CHCl₃:MeOH:H₂O (7:13:8).

Thin layer chromatographic (TLC)was performed on Uniplate HPTLC-HLF normal phase silica gel (150microns) Analtech while preparative thin-layer chromatography was carried out on pre-coated thin-layer chromatographic plates silica gel GF (2000microns). The plates were visualized with UV-lamp (Chromato-Vue Model CC-20) and sprayed with appropriate spray reagents to detect the spots.

GC-MS A Hewlett Packard HP 5890 CC was interfaced with a quadrupole mass spectrometer (MS) (HP5970). OV351 and BP1 fused silica columns (25 x 0.20mm i.d.) were used with helium as carrier gas (0.5ml/min). The temperature was programmed from 70°C to 200°C (50°Clmin.). MS operating parameterwere: ionization voltage- 700V, scan rate -1100 amu/sec, electron multiplier energy - 1600V, and ion source temperature 250°C. On-line acquisition and computation of mass spectra data were performed using Hewlett Packard HP 9827 computer equipped with a disc memory (HP9145). The constituents were identified by comparison of their mass spectral data with the computer library

### Preparation of Test Material

Extracts of each plant parts, including roots, stem bark, leaves, fruits, or seeds, was prepared by either extracting 200g of the dried coarsely powdered plant using soxhlet extractors or macerating at room temperature overnight with shaking. The bulk extracts from each plant was then filtered and evaporated to dryness in vacuo using the rotary evaporator. The crude extracts were screened for biological activities using a battery of test systems to detect antifungal, antimalarial, antileishmanial and antitrypanosmal activities etc. Each plant extract was intially submitted for a particular test based on their ethnomedical usage. Bioassay-directed fractionation of the active extracts/fractions using a combination of chromatographic techniques; gel filtration by sephadex, droplet countercurrent chromatography (DCCC), and low-pressure liquid chromatography (Lobar), open column chromatography or preparative thin layer chromatography(PTLC) led to the isolation and characterization of biologically active compounds.

### Bioassay-Directed Fractionation Protocol

### Antifungal Tests-Bioautographic (Reference example)

A method similar to that of Homans and Fuchs (1970) was employed. This technique involves direct spraying of thin layer chromatograms with conidial suspensions of a test organism. About 100µg of extract was spotted on silica gel TLC plates and developed with suitable solvent system. Dried plates were separately sprayed with either a spore suspension of *Cladosporium cucumerinum,* and subsequent plates with spore suspensions of *Cladosporium carrionii, Cladosporium cladosporioides, Cladosporium tennuisimum* (ATCC 623337) and *Fonsecaea pedrosoi* (ATCC 52593), in some cases to determine the spectrum of activity. The plates were then incubated in sealed humid chambers at room temperature for four days in the dark. Antifungal activity was manifested by the appearance of a white spot, corresponding to the position of the active compound, surrounded by a grey-black fungal growth all over the plates

To illustrate the bioassay-directed fractionation protocol, an example was drawn from the fractionation of rhizome extract of *Aframomum danellii* which showed strong antifungal activity. The powdered rhizome *of A. danellii* (200g) was continously extracted with petroleum ether (bp 40-60°C) to exhaustion in as soxhlet extractor. The petroleum ether extracts when combined and concentrated to dryness *in vacuo* gave a reddish brown residue (11.41g). The air dried marc from the above extract was again extracted with ethanol in a soxhlet extractor, extracts were concentrated to dryness under reduce pressure using the rotary evaporator at 40°C. Both extracts were tested for antifungal activity using bioautography (TLC-bioassay) as described above. Screening for antifungal activity showed that the petroleum ether extract of *A*. *danellii* were fungicidal against *Cladosporium cucumerinum* on a TLC bioassay

The active petroleum spirit extract (3g) was subjected to bioassay-directed fractionation by flash chromatography with a gradient elution of 1% methanol in chloroform. Fractions (20ml) were collected and pooled on the basis of their TLC profiles. Low pressure liquid chromatography (Lobar) separation of the most antifungal fraction (400mg) was performed in two successive separations on Lichro-prep silica gel 60 (40-60um, Merck) eluted first with petroleum spiritlethylacetate (6:1) and then with toluene/ethylacetate (95:5). This yielded major two UV active compounds of which only one inhibited the growth of *Cladosporium cucumerinum* spores at a minimum concentration of 0.5 µg. The flow chart of this procedure is shown in Fig 2.

### In Vitro Antileishmanial Activity

An *in vitro* radiorespirometric microtest (RAM) technique was used to evaluate the extracts/fractions or pure compounds for possible antileishmanial activity. This method, as already noted, relies on drug inhibition of parasite production of ¹⁴CO₂ from a battery of ¹⁴C-substrates by promastigotes to detect drug-mediated parasite damage at low drug concentration within a short time. The test is quantitative, rapid, consistent, and is conducted in serum-free medium in which prior adaptation is not necessary to cultivate the so-called "difficult to grow" species.

### Leishmania species/strains:

A clinical isolate of visceral *Leishmania (Leishmania) chagasi,* MHOM/BR/84/BA-1 3, was used for this study. This isolate was selected because sensitivity to SbV was previously determined using RAM. MHOM/BR/84/BA-13 is sensitive to Pentostam®, sodium antimony gluconate, at 6µg ml⁻¹ Sb (20µg ml⁻¹ drug); and to Glucantime®, N-methylglucamine antimonate, at 80µg ml⁻¹ Sb (286µg ml⁻¹drug).

The ¹⁴C-labeled substrates as shown in Table 1 of Fig. 6, are (numerical codes given in the x-axis of Figs. 2-5) ¹⁴C-substrates: (3) L-aspartic acid (4-¹⁴C); (7) glycine (U-¹⁴C); (10) L-leucine (1-¹⁴C); (13) L-omithine (1-¹⁴C); (25) D-galactose (1-¹⁴C); (28) D-mannose (1-¹⁴C); (44) succinic acid (1,4-¹⁴C); (46) Na-n-butyric acid (1-¹⁴C). All ¹⁴C-substrates were selected with specific activities as close to 40 mCi mM⁻¹ per carbon atom as obtainable from commercial sources. The quantitative promastigote growth inhibition assay was used as a guide to identify isolates exhibiting antileishmanial activity.

### RAM Drug Test Procedure:

The procedure was conducted as previously described (Jackson et al., 1989, 1990). Promastigotes were maintained in log phase growth for 3 successive transfers (48-72 h apart) prior to radiorespirometric (RAM) testing. Test samples (or PBSS, 0.1 M phosphate-buffered balanced salt solution, plus drug solvent, DMSO, for parallel control cultures) was added 24 h after the third promastigote transfer to fresh growth medium. Incubation in the presence of plant samples was continued for 96 additional hours while the parasites remained in mid-log phase growth. The test sample was tested at 50µg ml⁻¹. Drug sensitivity or resistance was based on ¹⁴C-substrate(s) (listed in Table 1) for which ¹⁴CO₂ release was decreased for drug-treated parasites compared to parallel tests of phosphate-buffered balanced salt solution and vehicle (PBSS+DMSO) controls. Each experiment consisted of parallel: (a) duplicate tests of drug-treated parasites; plus (b) duplicate tests of drug vehicle control-treated parasites; plus (c) one "nonbiological" sterility control. The nonbiological control consisted of each ¹⁴C-substrate (one substrate per microtiter tray well), and PBSS (the same PBSS batch used to wash, to suspend the parasites, and to make drug solution). Since there were no parasites in the nonbiological control, any ¹⁴CO₂ detected was attributed either to biologic contamination (or, less likely, chemical contamination) of the ¹⁴C-substrates resulting in breakdown of the ¹⁴C-substrates. If radioactivity above background (10 dpm) was detected in the nonbiological control, the suspect solution(s) was replaced and the experiment was repeated.

### Promastigote viability was assessed by morphological criteria such as flagellar mobility and parasite morphology.

The powdered rhizome (208g) of *Aframomum daniellii* (reference example) *was* soxhlet extracted with petroleum ether (b.p 40-60 °C) and ethanol. The petroleum ether extract of A. *daniellii* which showed pronounced antileishmanial activity was further investigated to isolate the active constituents The active petroleum ether extract (3g) was subjected to bioassay-directed fractionation by flash chromatography with a gradient of 1% methanol in chloroform as described earlier. Fractions (20ml) were collected and pooled on the basis of their TLC profiles.

The resulting four fractions were then screened for antileishmanial activity and the most active fraction (Fr.3) was analyzed by GC-MS to access its purity and identification as shown in Figs. 2A-C. Low pressure liquid chromatography (Lobar) separation of the antileishmanial fraction was performed as described above in two successive separations using Lichro-prep silica gel 60 (40-60*µ*m, Merck) This yielded the same Labda-8(17),12-diene-15,16-dial identified above as the antileishmnaial compound. The identity was established based on spectra analysis and comparison with the literature data.

### Bioassay-directed fractionation of Napoleonaea imperialis P. Beauv. (Fam Lecythida Caec) seed extract.

The effect of *Napoleonaea imperialis* seed extract and isolated pure compounds on promatigotes were assessed by *in vitro* Radiorespirometric Microtest (RAM) technique as described above.

The powdered seed of *Napoleonaea imperialis* was soxhlet extracted with solvents of increasing order of polarity in three batches, starting with hexane (48 h), chloroform (48 h), ethyl acetate (48 h) and methanol (48 h). Each extract was concentrated to dryness in vacuo using the rotary evaporator at 40°C. The promastigote growth assay was used to identify fractions exhibiting antileishmanial activity. The methanol extract was the most active fraction. A portion of the methanol extract (20 g) was first partitioned between chloroform-methanol-water mixture (2:2:1, 1000ml) to yield a saponin-enriched lower organic layer which was concentrated to dryness *in vacuo* and lyophilized. Both factions showed antileishmanial activity were subjected to fractionation by column chromatography on silica gel with a gradient elution of chloroform methanol. Preparative low pressure Liquid chromatography of the antileishmanial fraction was performed on silica gel with CM (1:), yielding an antileishmanial substance , consisting of a mixture of three saponins.

Screening for antileishmanial activity showed that the methanol extract of N *imperialis* showed inhibitory effects against the growth of *Leishmania* promatigotes. A second method of generating active components from *N imperialis* was carried as follows. A portion of the methanol extract (11.35g)was dissolved in minimum amount of methanol and subsequently precipitated with diethyl ether, to yield a 59% of saponin-rich portion(6.73g) and 4.54g % of non-saponin portions. Both fractions were submitted for antileishmanial screening. The saponin portion showed remarkably more antileishmanial activity than the non-saponin portion. The active saponin-rich fraction (6.03g) was subjected to fractionation by column chromatography on silica gel with a gradient elution of chloroform methanol. Fractions (20ml) were collected and pooled on the basis of their TLC profiles. The resulting fractions (1-7*) were then screened for antileishmanial activity. Fraction 13-15 (1.48g) eluted with chloroform:methanol (5:2) gave the most active fraction and subsequent re-chromatographed on silica gel column eluted with chloroform:methanol (3:2) to yield several sub-fractions. The residues of the fractions possessing the major activity were re-chromatographed over silica gel by using eluents comprising chloroform:methanol to which increasing amounts of methanol were added. After screening these fractions for antileishmanial activity, sub-fraction 36-51 (352.6g). was found to be the most active and was subsequently purified by low pressure preparative liquid chromatography using on Rp-8 lobar column eluted with methanol: water(7:3).This yielded three active fraction; imperialiside (A-C) (NI-3 (152mg), NI-4 (30mg) & NI-5 (102mg)).

### Bioassay-directed fractionation of Eupatorium odoratum P. Beav. (Fam. Lecythidacaea) leaf extract.

By the same process used, the powdered seeds of *Eupatorium odoratum* was successively extracted with petroleum ether bp (40-60 °C) (48 h) and methanol (48 h) using soxhlet extractor. Each extract was concentrated to dryness *in vacuo* using the rotary evaporator at 40 °C. Both extract showed antileishmanial activity with the methanol extract showing greater inhibition of the growth of promastigotes. A portion of the methanol extract (20.0g) was first partitioned between chloroform-methanol-water mixture (2:2:1) to yield a lower organic layer and a more polar aqueous layer. Both fractions were concentrated to dryness *in vacuo* and lyophilized. The more active organic fraction (8.10g)was subjected to further bioassay-directed fractionation by column chromatography on silica gel. The column was eluted with chloroform:methanol (19:1; 1:3, ethyl acetate, ethylacetate:MeOH 17:1). Fractions (20ml) were collected and pooled on the basis of their TLC profiles. The monitoring of the fractions was carried out with TLC aluminum sheet silica gel 60-F254 in solvent system I and II. After screening these fractions for antileishmanial activity, fraction 60-104 was found to be the most active and was subsequently purified by combination of gel filtration on Sephadex LH-20 column (2.0 X 50 cm) LC-No. 5, Fig. 3. and preparative thin layer chromatography, leading to the identification of Sakuranetin as the antileishmanial compound. The structure of sakuranetin (II) was established by GC-MS and Nuclear magnetic resonance spectroscopes and by comparison with published data, as shown in the following formula:

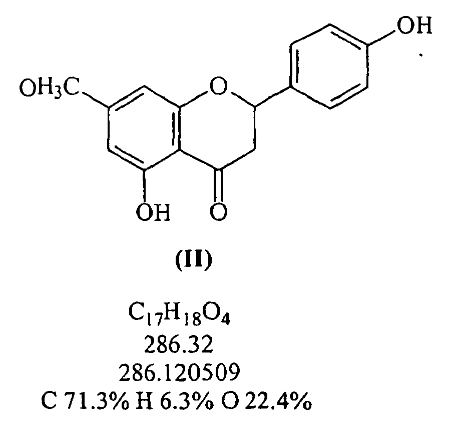

### Antimalarial Bioassay

The *in vitro* antimalarial assays were performed by using a modification of the semi-automated microdilution technique described earlier (Desjardins *et al.,* 1979, Milhous *et al.,* 1985). Two *Plasmodium falciparum* malaria parasite clones, designated Indochina (W-2) and Sierra Leone (D-6), were utilized in susceptibility testing. The W-2 clone is resistant to chloroquine, pyrimethamine, sulfadoxine, and quinine, and the D-6 clone is resistant to mefloquine.

The test extracts/compound, was dissolved in DMSO and serially diluted using malarial growth medium. Drug-induced reduction in uptake of titriated hypoxanthine was used as an index of inhibition of parasite growth.

### In Vivo Antileishmanial Activity

The *in vivo* antileishmanial activity was determined by administering various doses of the *Napoleonaea imperialis* extracted to golden hamsters and determining the effect on laboratory-induced visceral and cutaneous leishmanial of the animals. For this assay, the compounds were tested against *Leishmania (Leishmania) donovani,* MHOM/SD/43/Khartoum, a causative organism of kala azar or visceral leishmaniasis, and *Leishmania (Viannia) panamensis,* MHOM/PA/83/WR539, an etiological agent of simple cutaneous leishmaniasis. Imperiside A was tested in each *in vivo* leishmanial model by the oral, intramuscular, and subcutaneous routes of administration.

### Cytosensor Microphysiometer System

The rate at which cell excrete acids into their environment is closely linked to the rate which they convert food to energy, i.e. metabolic rate. The Cytosensor Microphysiometer System (CMS) measures the rate at which cells acidify their immediate environment. The CMS monitors these metabolic changes as changes in the rate of cellular acidification. In this way, the system provides a real-time, noninvasive means of measuring cellular responses to a wide variety of agents (McConnell *et al.,* 1992).

Extracts of *Napoleonaea imperialis* seeds were tested for antileishmanial activity *in vitro* using CMS. Promastigote leishmanial forms were exposed to N. *imperialis* in the chemically defined, serum-free medium (Jackson *et al.,* 1989) for 17.5 h during logarithmic growth phase. To prepare cells for CMS, the non-adherent cell protocol was utilized. Briefly, the cells were centrifugally concentrated, counted by hemacytometer, and re-suspended in 0.2% low temperature agarose in balanced salt solution. Leishmanial promastigotes, a 10µl suspension containing 1-2 X 10⁶ cells in agarose, were placed in each of 8 Cytosensor flow-chambers and the low-buffer formulation of RPMI medium (pH 7.4, Molecular Devices Corporation) was pumped over the cells. The repetitive pump cycle time was 2.0 min (88 sec of medium flow followed by 32 sec of pump off). During the 32 sec the peristaltic pump was not operating, the rate of leishmanial acidification of RPMI medium in each of 8 separate cell chambers was measured. Acidification rates during the two-min cycle resulted in less than 0.1 pH unit change and were not detrimental to the leishmanial cells. The CMS leishmanial acidification rates (representative data given in Fig. 4) were relatively constant for each drug treatment concentration (6.3, 12.5, 50µg ml⁻¹) and vehicle control (0.6% DMSO) duplicate pair, tested in parallel simultaneously, over the 11 h observation period.

### DRUG DEVELOPMENT FOR PARASITIC PROTOZOA

Haskins Laboratories has focused on the nutrition and biochemistry of protozoa and the development of novel leads to chemotherapy of parasitic protozoa for >40 years. Research initially stressed nutritional and vitamin-requirement studies leading to development of chemically defined media for many free-living and parasitic protozoa. Recent studies have emphasized African trypanosomes, trichomonads, and the opportunistic pathogens *Cryptosporidium* and *Microsporidia.*

The biochemical studies surrounding drug development have centered on the synthesis and metabolism of polyamines in these organisms, with biochemical peculiarities in the protozoa serving as focal points for targeting antiparasitic agents.

Our study of polyamine synthesis in African trypanosomes led to the development of DFMO, an enzyme-activated inhibitor of ornithine decarboxylase, for clinical use in human trypanosomiasis (Bacchi et al., 1980; Bacchi & McCann, 1987; Sjoerdsma & Schechter, 1989). This agent was approved by the U.S. Food and Drug Administration in 1990 and has been used clinically in greater than 2,000 cases of West African disease with a 95% cure rate (Kuzoe, 1993; pers. commun.; WHO, 1995; van Nieuwenhove, 1992). The mechanism of DFMO action in part has been investigated extensively; our studies indicate it acts in trypanosomes by causing overproduction of S-adenosylmethionine (AdoMet), a precursor of spermidine (Yarlett & Bacchi, 1988a). Recent work indicates that the over synthesized AdoMet is rapidly used in the methylation of proteins and lipids (Bacchi et al., 1995; Goldberg et al., 1997), aiding in the blockage of cell division.

Polyamine synthesis in *T. vaginalis* was found to differ from mammalian cells in that AdoMet decarboxylase was lacking and that spermidine was obtained from conversion of exogenous spermine, taken up through an amine transport system, and then converted through a polyamine oxidase (Yarlett & Bacchi, 1994). Although putrescine was synthesized from arginine through the arginine dihydrolase pathway, this process also functions in part to generate ATP and to generate putrescine which is exchanged for spermine through an antiporter (Yarlett & Bacchi, 1988b; Yarlett & Bacchi, 1994; Yarlett et al., 1994, 1996). These findings indicate that *T. vaginalis* (and *T. foetus)* obtain polyamines by processes differing from mammalian cells (Yarlett et al., 1992, 1993; Bacchi & Yarlett, 1995). We are examining the effects of amine analogs for ability to enter through the polyamine transport system (Yarlett et al., 1992; Woster et al., 1993), and for amine oxidase inhibitors to reduce polyamine interconversion.

Our most recent work has also focused on two opportunistic pathogens frequently associated with chronic intractable diarrhea in AIDS, *Cryptosporidium* and *Microsporidia.* The diseases caused by these agents are presently incurable. Biochemical studies on the apicomplexan, *Cryptosporidium parvum,* have indicated that polyamine production is initiated by conversion of arginine to agmatine via arginine decarboxylase (ADC), an enzyme normally associated with plants. This enzyme has been partly purified and characterized from C. *parvum* (Keithly et al., 1997), and the efficacy of polyamine analogs and ADC inhibitors are now being assessed in C. *parvum* growth.

*Microsporidia* spp. are a group of parasites encompassing at least five genera. These organisms are thought to be protozoans on the basis of structural features of the zoospore. This group of organisms has been implicated in numerous pathological conditions including kerato conjunctivitis, brain lesions and intense diarrhea in immunosuppressed patients, especially those with AIDS (Wittner et al., 1993). We have begun studies with *Enterocytozoon cuniculi,* examining polyamine metabolism and the ability of polyamine analogs to inhibit growth of this intracellular intestinal parasite. Our critical observations indicate that two polyamine analogs 1,11-diethylnorspermine and 1,19-bis(ethylamino)5,10,15-triazanonadecane at 100mM sterilize the culture of this parasite, are without toxicity to the feeder layer RK13 cells (Coyle et al., 1996).

These studies plus the collaboration of the Haskins Laboratories group with >10 synthetic chemists at eight institutions (Aubum Univ., Cornell Univ., Johns Hopkins Univ., Roswell Park Cancer Inst., Tennessee State Univ., Univ. of Michigan, Univ. of Wisconsin, Wayne State Univ.) indicates the focus of the group on chemotherapy of protozoan-caused disease µg ml⁻¹ was used as reference standard and 0.1 ml DMSO as a control. The plates were incubated at 37°C and the diameter of zones of inhibition was measured across each well after 24 h. The MIC for bacteria was determined in trypticase soy broth to which were added serial 2-fold concentrations (0.025-200µg ml⁻¹) of Mannispirostan A. The tubes were inoculated in triplicate with 0.01 ml quantities of 6th broth cultures of the test isolates. The tubes were incubated at 37 °C for 24 h and examined spectrophotometrically at 530 nm. The lowest drug concentration that showed no turbidity was taken as the MIC. Streptomycin was used as the standard reference drug.

### RESULTS AND DISCUSSION

Rapid methods for screening, isolation, and characterization of antiprotozoal compounds are described. These methods utilize relatively small quantities of plant sample or compounds. By these methods, the major compounds identified from the antileishmanial study include labdane-dialfrom *Aframomum daniellii* (reference example), Mannispirostan A from *Dracaena mannii* and *D. arborea* (reference example)*,* Sakuranetin from *Eupatorium odorantum* and *Imperialisides* from *Napoleonaea imperialis;*

In an activity-directed investigation of the extracts of leaves, stem and rhizome of *Aframomum daniellii,* the petroleum ether extract of the rhizome of *A. daniellii* exhibited strong antifungal and antileishmanial activities, as shown in Fig. 1. Bioassay directed fractionation of this active fraction led to the isolation of a labda-8(17), 12-diene-15,16-dial (I), as shown in the following formula:

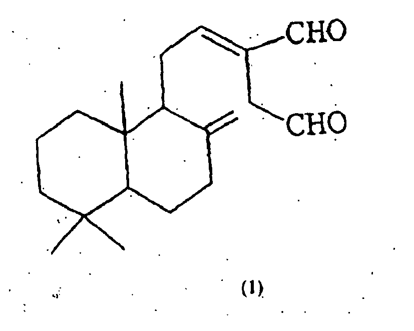

### Characterization of labda-8(17),12-diene-15,16-dial (Reference example)

TLC(SiO₂, Tol/EtoAC 6:1) Rf = 0.39. m.p 90- 92oC
UV λₘₐₓ CHCl₃ 240nm; IR Vₘₐₓ (KBr) 2940, 1730,
1680 and 1640 cm-1; DCI-MS m/z (rel.int.) 302(21),
287(11) 273(9), 258(18), 241(4), 231(4), 190(7), 177(10),161(8), 147(13), 137(100), 123(54),109(29), 95(45),91(31), 83(66), 69(52), 55(30).

Labda-8(17),12-diene-15,16-dial showed strong absorption at 1680 cm⁻¹ which is assigned as an β-unsaturated carbonyl group. The UV absorption maximum at 240nm supported this assignment. DCI-MS showed a molecular ion peak of m/z 302 and base peak of 137, suggesting a molecular formula of C₂₀H₃₀O₂. There is good agreement between the above spectroscopic data and those reported for labdane dialdehyde (Kimbu et al, 1979 and Hideji et al 1980) The presence of this compound is being reported for the first time in the genus *Aframomum.* However, Labda-8(17),12-diene-15,16-dial was first reported in *Alpinia speciosa* (Itokawa et al, 1980) and related diterpene (E)-8b,17-epoxy-12-ene-15,16-dial from A. *daniellii.The* bioassay-directed chromatographic separation of Labda-8(17),12-diene-15,16-dial from the petroleum ether extract monitored by gas chromatographic analysis are shown in Figs. 2A-C.

The antifungal activity of extracts of this plants was originally detected by direct spraying of TLC plates with a spore suspension of the test fungus *Cladosporium cucumerinum.* A clearly visible inhibition zone, even at the lowest concentration of 0.5µg was observed after using labdane-dial, as shown in Fig. 1. The most striking result was obtained with Labda-8(17),12-diene-15,16-dial which inhibited the *Leishmania* promastigotes at a concentration of 50µg or less.

The result of the RAM test showed that the labdane-dial completely inhibited the growth of the leishmania strain at a dose of 50µg/ml. The test compounds also inhibited the catabolism of various substrates. The results of the RAM test for leishmanial parasites are shown in Figs. 2D. E. After a 96hr incubation with labdane-dial, no live parasites were observed in culture and RAM respiratory rates for all ¹⁴C-substrates reflect this lack of parasite viability. The metabolic rate for every ¹⁴C-substrate by the labdane-dial -treated parasites is near zero (solid pink bars). The drug-treated results are in sharp contrast to the vehicle control (0.6% DMSO) treated promastigote ¹⁴C-substrate catabolism, which show high respiratory rates during the 30 min test period (solid green bars).

Most of our preliminary work utilized radiorespirometric in vitro methodology (Jackson, et al., 1989; Jackson et al., 1990). Radiorespirometry is based on drug inhibition of parasite catabolism of a variety of diverse, simple ¹⁴C-substrates (simple sugars, amino acids, amines, fatty acid precursors, nucleic acid precursors, etc.) to ¹⁴CO₂. We have data on >55 different ¹⁴C-substrates. Thus, radiorespirometry offers the advantage of checking specific drug inhibition of a variety of physiologic pathways simultaneously, depending on the type and number of ¹⁴C-substrates utilized for each drug test. This compound being a lipophilic diterpene dialdehyde would be expected to associate strongly with membrane lipids.

Radiorespirometry is a very sensitive method, effective in detection of drug activities to <1 mg/ml using cultured promastigotes. Controls are two-fold, one non-biologic control in which parasites are omitted from the test to verify sterility of the ¹⁴C-substrates; and parallel cultivated promastigotes treated only with the solvent solution (containing no drug) as a drug "vehicle" control. However, use of radioactive material has some very pronounced disadvantages, biohazard and cost.

Labda-8(17),12-diene-15,16-dial also inhibited the in vitro growth of both a chloroquine-susceptible (D6) and a chloroquine-resistant (D2) strain of Plasmodium falciparum in a [³H]hyproxanthine uptake assay, as shown in Table 2.

In this assay, the Labda-8(17),12-diene-15,16-dial treatment resulted in an IC50 value of 280.18 ng/ml for the W-2 clone, and 96.66 nglml for the D-6 Plasmodium falciparum clone. This compound has been known to exhibit strong antifungal activity.

### Molecular model

Molecular representation of the Labda-8(17),12-diene-15,16-dial (reference example) showing ball and stick model, space-filling model, total electron density surface including Lumo and Homo surfaces as well as isopotential surfacaes at both -10 kcal/mol and -5.0 kcal/mol are shown in Fig. 2F. The figure revealed several interesting electronic surface of the molecule such as total electron density, location of the most nucleophilic and electrophilic sites, sites for nucleophilic and electrophilic attacks and molecular recognition patterns for receptor binding.

### Napoleonaea imperialis (Lecythidaceae)

The ethylacetate and alcoholic extracts of *Napoleona imperialis* seeds were effective *in vitro* at concentration of 50µg/ml or less using visceral *Leishmania* isolates as shown in Fig. 3. Bioassay-directed chromatographic fractionation of the active extracts led to the isolation of three promising antileishmanial compounds tentatively identified as *imperialisides(A-C).* The extracts and pure compounds showed significant activity *in vivo* (suppression of lesion size & *L.donovani* units) on hamster challenged with cutaneous or visceral *Leishmania* isolates as shown in Table 2. There was no apparent toxicities during the experiment. The results of the activity of the *Napoleonaea imperialis* administered through the intra-muscular route to hamsters infected with cutaneous *L. panamensis* represent an example of dose-dependent *in vivo* activity of the compound. At a dose of 104 mg kg⁻¹ total dose (equivalent to 26 mg kg⁻¹ per day) of the *Napoleonaea imperialis,* administered by intramuscular route twice a day for 4 days, the test substance produced a 73% inhibition of lesion caused by *L. panamensis* in hamsters. A dose of 52 mg kg⁻¹ (13 mg kg⁻¹ per day) by the same regimen gave a 51 % reduction of the lesion area, and at a dose of 13 mg kg⁻¹ (3.25 mg kg⁻¹ per day) 7% reduction of the lesion area was observed.

The results of antileishmanial activity shown above (RAMs and *in vivo* method) corroborated the results from cytosensor. Figs. 4A-4C illustrate *Cytosensor Microphysiometer* of promatigote incubated in the presence of 50mg of *N. imperialis* per ml or medium alone. Confirmed antileishmanial activity was observed using cytosensor, which is a new nonradioisotopic microchip-based method.

Furthermore the results using the Cytosensor as shown in Fig. 4 agree well with visual observation of the parasites by light microscopy and the growth inhibition curve, The vehicle control parasites, manifest the typical spindle-shaped monoflagellate form of leishmanial promastigotes. Cell density of the control parasites in culture was 5 X 10⁷ ml⁻¹. At 50µg ml⁻¹ drug, no intact parasites are visible, only hollow parasite membranes, with no cytoplasm. Likewise, an IC₅₀ of approximately 10µg ml⁻¹ was observed for the growth inhibition data, Maximum achievable serum level for SbV drugs, current "drugs-of-choice" for antileishmanial therapy, has been determined to be 20µg ml⁻¹ 1-2 h post-administration (references reviewed in Jackson, *et al.,* 1989, 1990).

Comparative analyses of the polar extracts from *Napoleonaea imperialis* demonstrated that the *imperialiside* analogues are the major biologically active components with *imperialiside* A & B being the most active compounds. These biological effects can perhaps explain the traditional use of the these plant species in treating different skin diseases.

### Sakuranetin from Eupatorium odorantum

The methanol extracts of E. *odoratum* exhibited very strong antileishmanial activity. Bioassay guided fractionation of this extract yielded a mixture of yellowish compounds. The activity was concentrated in the flavonoid fractions which yielded 4 flavanoids. The strongest antileishmanial activity were observed with sakurenatin (II), as shown in Fig. 5.

Available literature indicates that no previous antileishmanial study has been carried out on *E. odoratum* and there is scanty reports on the chemical constituents of the local variety of this plant which appears a new comer in the West African vegetation. At a concentration of 50 *µ*g/ml, total inhibition of promastigote growth occurred as shown in Figs. 5A and 5B. About 100% inhibition of promatigote growth was observed with both petroleum ether and methanol extracts. Partitioning the methanol extract into organic and aqueous fractions, the organic fraction showed greater inhibitory activity than the aqueous fraction as shown as Figs. 5C and D. The methanol extract displayed maximum inhibitory activity in the Radiorespirometric microtest when compared with the petroleum ether and water extracts At this concentration, about 95% of the promastigotes exhibited abnormal round morphology. Further bioassay-guided chromatographic fractionation of the organic fraction using column chromatography on silica gel eluted with CM(19:1; 9:1), EtOAc, EtOAC:MeOH(17:1) yeilded five sub-fraction with varying activity, as shown in Figs. 5G and 5H. Sub-fraction eluted with EtOAC, as shown in Fig. 5E, showed the greatest inhibitory activity. The most active antileishmanial constituents of E. *odoratum* was isolated from these two fractions. The pure compound was identified as sakuranetin by spectra analysis as well as Co-TLC with authentic samples.

In recent years many effort have rationalize the bioactivity and the importance of flavonoids (Middleton and Kandaswami 1994). One of such activity is the antileishmanial activity exhibited by sakurenatin isolated from *E*. *odoratm.* Very few flavonoids have been reported to process antileishmanial activity.

Sakuranetin was isolated from the most active fraction while Lupeol, a-amyrin, betuletol, 3,5,7,3'-tetra-O-methyl quercetagetin, quercetin and two flavonoid glycosides based on sakuranetin and isosakuranetin moieties were isolated from inactive extract/fractions. Sakuranetin being a flavonoid belongs to a class of natural products generally known to be non-toxic. These findings suggest that sakuranetin in concentration that are nontoxic to the host cell may exhibit a strong antileishmanial activity *in vivo* and that appropriate substituted flavonone having basic sakuranetin skeleton might provide new class of antileishmanial drugs.

Because antiprotozoal and antifungal activities are frequently associated with the same or chemically similar compounds, we considered it probable that natural products that show remarkable antifungal activity such as, *imperialiside,* labdane-dial and Sakuranetin would have antiprotozoal activity.

Sakuranetin also inhibited the *in vitro* growth of both a chloroquine-susceptible (D-6) and a chloroquine-resistant (W-2) strain of *Plasmodium falciparum* in a [3H]hypoxanthine uptake assay, as shown in Table 2, with IC₅₀ values of 169.95ng ml⁻¹ for the W-2 clone, and 123.88ng ml⁻¹ for the D-6. These results demonstrate that Sakuranetin exhibits potent antimalarial activity and might be developed into a new antimalarial drug.

In Africa, traditional medicine with herbal treatment has a long history and is used routinely in medical care (Hartey, 1941; Feierman, 1981; Assi & Guinko, 1991), however only a few reports document activity of plant extracts against African trypanosomes. One study demonstrated activity of extracts of *Khaya* spp seeds (West African hardwoods) *in vitro* vs. *T. b. brucei* (Owolabi et al., 1990). The active agents were a group of furanodlimonoids related to quassinods, plant products found to block protein synthesis (Kirby et al., 1989). Another report indicated that gossypol, a quassinoid, blocked respiration and destroyed *T. b. brucei* blood forms *in vitro* at micromolar concentrations (Eid et al. 1988). lgweh and Onabanjo (1989) cured mice infected with *T. b. brucei* using aqueous root extracts of *Annona senegalensis.* Recently, a series of studies by Freiburghaus et al. (1996a, 1996b, 1997) evaluated extracts of traditional medicinal plants from Uganda, Tanzania and the Ivory Coast, against *T. b. rhodesiense* blood forms *in vitro.* Of those tested, 42 of 310 extracts (13.5%) were found to have significant growth inhibitory activity at 10 mg/ml or less. The active agents generally had modest selectivity indicies, as compared to commercially available agents, but, overall, these studies confirmed the potential for ethnobotanically selected plants as potential sources of agents against sleeping sickness (Freiburghaus et al., 1996a, 1996b, 1997).

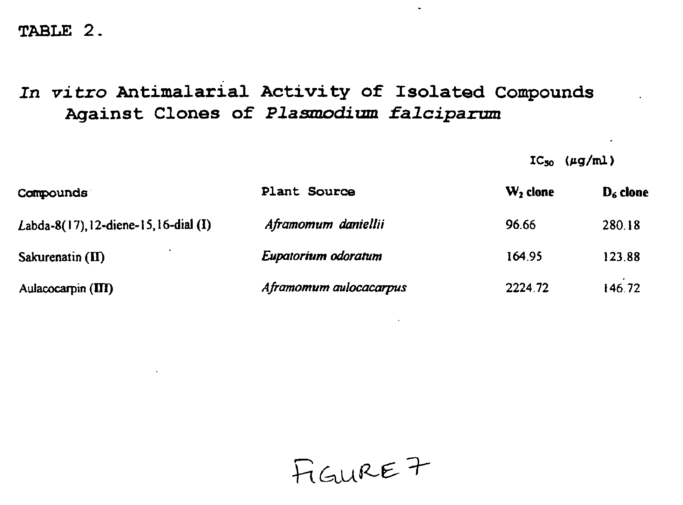

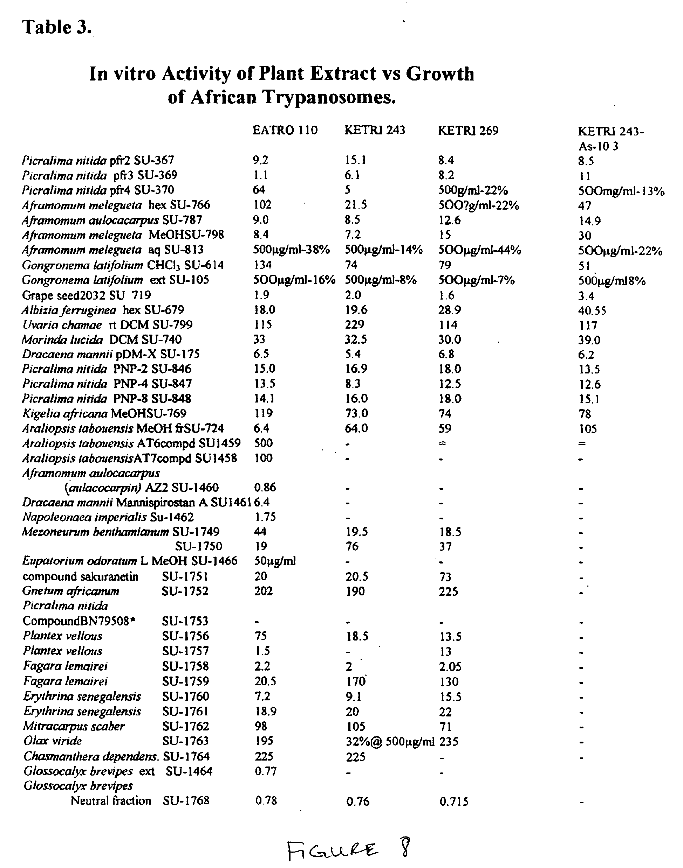

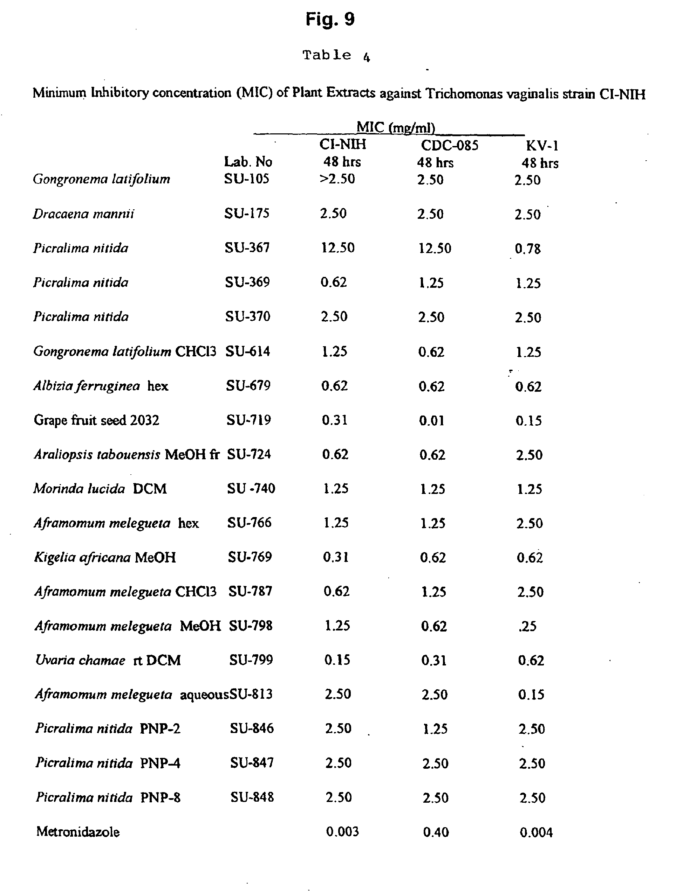

The *in vitro* activity of 39 plant extracts was tested against four strains of animal or human-pathogenic African trypanosomes, and three strains of mammalian-pathogenic *Trichomonas* spp. The trypanosomes studied were *Trypanosoma brucei brucei Lab* 110 EATRO, which is pathogenic to cattle and other livestock, and several strains of *Trypanosoma brucei rhodesiense,* a parasite of humans, domestic and wild animals. Strains of *T. b. rhodesiense* included drug resistant clinical isolates KETRI 243 and 269 and KETRI 243 As-10-3, a highly melarsen- and diamidine-resistant clone of KETRI 243. The 39 extracts were tested in an *in vitro* screen using a semi-defined medium for growth of bloodstream trypomastigotes at 37° C (Hirumi & Hirumi, 1989) to determine IC₅₀ values (Bacchi et al., 1996). Using a cutoff of 100 µg/ml, 28 of the 39 extracts consistently gave IC₅₀ values in the active range, as shown in Table 3 of Fig. 7. Of these, 10 had IC₅₀ values at or below 10µg/ml and were considered sufficiently active to warrant testing of more purified extracts. Of the four secondary extracts supplied, one, aulacocarpin (III) (SU1460), derived from the primary extract SU787 of *A.aulacocarpus* featured a 10-15 fold increase in activity. SU787 had IC₅₀ values of 8.5-14.9 mg/ml, while the value for SU1460 was 0.86 mg/ml. Eight additional primary extracts were also tested in the trypanosome screen (Table 3). Of these, SU 1462 from *Napoleonaea imperialis* and SU 1464 from *Glossocalyx brevipes* were highly active (IC₅₀~1 mg/ml) and warrant further study. Aulacocarpin (III) is an extract from *Aframonum aulacocarpus* and has the following formula: 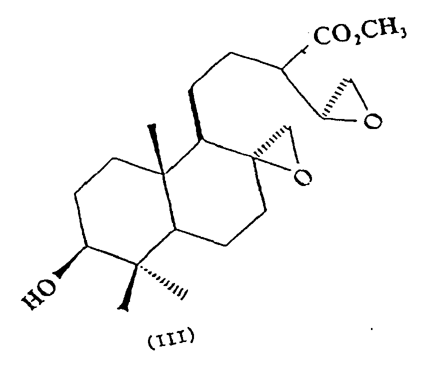 The trichomonad screen consisted of two human pathogenic *Trichomonas vaginalis* strains and a livestock parasite *Tritrichomonas foetus.* The *T. vaginalis* isolates include a metronidazole sensitive isolate (C1-NIH: ATCC 30001) and a strain highly resistant to metronidazole (CDC-085: ATCC 50143). The screening procedure used is that of Meingassner et al. (1978) and determines the minimal inhibitory concentration (MIC) in mg/ml needed to completely inhibit growth. Table 4 of Fig. 9 details data from the initial group of 19 primary extracts. Of these, seven had MIC values of 1 mg/ml for all three isolates and were considered of interest for further study. Some fractions from the above active extracts were tested along with new extracts. The results show that the most active extract in this group was *Glossocalyx brevipes* which had an MIC value of 0.0125 mg/ml for each isolate and was the most potent of the primary extracts tested thus far.

The active plant extracts in each screen are listed in Tables 3 and 4 (many of the extracts tested are reference examples). The results are reported on the basis of MIC levels (< 1 mg/ml) for trichomonad screens and IC₅₀ values (</ =10 mg/ml) for trypanosomal screens. Although many of the extracts were most active only against one group of organisms, six extracts had significant activity against both groups. These were SU369, 719, 724, 787,1464 and 1465. Of these, SU719 and 1464 appeared to be most potent in both screens.

Although large-scale testing of plant extracts for activity against protozoan parasites is largely lacking (Wright & Phillipson, 1990) recent evaluation of African medicinal plants vs. *T. b. rhodesiense* has given some encouraging results (Freiburghaus et al. 1996a, 1996b, 1997). In these studies crude extracts were considered to have promising activity in an *in vitro* screen against blood forms if IC₅₀ values were at or below 10 mg/ml. In the above trypanosome screen 13 of 27 plant extracts had such activity while two (SU719 and 1464) had IC₅₀ values at or below 1 mg/ml. Further studies will need to examine the selectivity of active extracts, i.e. the maximum tolerated concentrations by mammalian cell lines vs. the IC₅₀ or MIC values. If the selectivity data is favorable, further purification of the active principles and animal testing would be the logical next steps in the exploration of these extracts.

An example of ethnobotanical enhanced activity is observed in the genus *Aframomum.* The plant, A*framomum danielli* (reference example)*, was* tested *in vitro* and found to be very active against *Leishmania (Leishmania) chagasi.* Using the leishmanial *in vitro* as radiorespirometric diorespirometric bioassay the active compound was purified and its structure determined as labda-8(17),12-diene-15,16-dial (1). A related species, *Aframomum meleguata* (reference example) showed moderate activity *against Trypanosoma brucei in vitro* IC₅₀ 9.0 mg/ml. However, a third plant species, *Aframomum aulocacarpus,* contains aframodial which showed activity within the highly active drug range, IC₅₀ 0.86 mg/ml, a 10-11-fold increase in activity Aframodial has since been shown to be a nontoxic broad spectrum antifungal agent (2)Morita, H and Itokawa H., Planta medica, 54, 117 (1988)The structural modifications in active antiparasitic with these botanical species changes are in progress. Numerous similarities in leishmanial and trypanosomal lipid uptake and metabolism may explain common natural product drug susceptibility. Protozoan diseases, including leishmaniases, offer little commercial drug development incentive, thus, the readily available, cheaper, oral and less toxic traditional medicined frequently prevail in developing countries.

As can be seen from the test results, compounds I-III surprisingly exhibit potent activity against *Leishmania (Leishmania) chagasi* the cause of cutaneous leishmaniasis as well as exhibiting antimalarial effects. This finding strongly supports the tremendous potential that exists in the exploration traditional remedies for lead compounds in the development of new anti-protozoal drugs.

While the claimed invention has been described in detail and with reference to specific embodiments thereof, it will be apparent to one of ordinary skill in the art that various changes and modifications can be made to the claimed invention without departing from the scope thereof.

## Claims

1. Use of an extract from Eupatorium odoratum for the manufacture of an anti-leishmanial or anti-malarial medicament.

2. Use according to claim 1, wherein the extract includes sakuranetin (compound II), extracted therefrom.

3. Use of an extract from Glossocalyx brevipes for the manufacture of an anti-trichomonal medicament.

4. Use according claim 3, wherein the extract includes extract SU-1464, extracted therefrom.

5. Use of an extract from Aframomum aulacocarpus for the manufacture of an anti-malarial or anti-trypanosomal medicament.

6. Use according to claim 5, wherein the extract includes aulacocarpin (compound III), extracted thereform.

7. Use according to any one of claims 1 to 5, wherein said extract is obtained from the roots, stem bark, leaves, fruits or seeds from said plants.

8. Use of an extract from Napoleonaea imperialis for the manufacture of an anti-leishmanial or anti-trypanosomal medicament.

9. Use according to claim 8, wherein said extract is obtained from the powdered seeds of Napoleonaea imperialis.

10. Use according to any one of the preceding claims, wherein said use involves a topical composition comprising an extract as defined in any of the preceding claims in a topical carrier.

11. Use according to any one of the preceding claims, wherein said use involves an oral composition comprising an extract as defined in any of the preceding claims in an oral carrier.

12. Use according to any one of the preceding claims, wherein said use involves an intravenous composition comprising an extract as defined in any of the preceding claims in an intravenous carrier.

## Patentansprüche

1. Verwendung eines Extrakts von Eupatorium odoratum für die Herstellung eines Leishmanien- oder Malariamedikaments.

2. Verwendung nach Anspruch 1, wobei der Extrakt Sakuranetin (Verbindung II), das daraus extrahiert ist, enthält.

3. Verwendung eines Extrakts von Glossocalyx brevipes für die Herstellung eines Trichomonadenmedikaments.

4. Verwendung nach Anspruch 3, wobei der Extrakt SU-1464 Extrakt, der daraus extrahiert ist, enthält.

5. Verwendung eines Extrakts von Aframomum aulacocarpus für die Herstellung eines Malaria- oder Trypanosomenmedikaments.

6. Verwendung nach Anspruch 5, wobei der Extrakt Aulacocapin (Verbindung III), das daraus extrahiert ist, enthält.

7. Verwendung nach einem der Ansprüche 1-5, wobei der Extrakt aus den Wurzeln, der Stammrinde, den Blättern, Früchten oder Samen der Pflanzen erhalten wird.

8. Verwendung eines Extrakts von Napoleonaea imperialis für die Herstellung eines Leishmanien- oder Trypanosomenmedikaments.

9. Verwendung nach Anspruch 8, wobei der Extrakt aus den gemahlenen Samen von Napoleonaea imperialis erhalten wird.

10. Verwendung nach einem der vorhergehenden Ansprüche, wobei die Verwendung eine topische Zusammensetzung involviert umfassend einen Extrakt, wie in irgendeinem der vorhergehenden Ansprüche definiert, in einem topischen Träger.

11. Verwendung nach einem der vorhergehenden Ansprüche, wobei die Verwendung eine orale Zusammensetzung involviert umfassend einen Extrakt, wie in irgendeinem der vorhergehenden Ansprüche definiert, in einem oralen Träger.

12. Verwendung nach einem der vorhergehenden Ansprüche, wobei die Verwendung eine intravenöse Zusammensetzung involviert umfassend einen Extrakt, wie in irgendeinem der vorhergehenden Ansprüche definiert, in einem intravenösen Träger.

## Revendications

1. Utilisation d'un extrait d'Eupatorium odoratum dans la fabrication d'un médicament antileishmania ou antipaludique.

2. Utilisation selon la revendication 1, où l'extrait inclut de la sakuranétine (composé II) extraite de celui-ci.

3. Utilisation d'un extrait de Glossocalyx brevipes dans la fabrication d'un médicament antitrichomonas.

4. Utilisation selon la revendication 3, où l'extrait inclut l'extrait SU-1464, extrait de celui-ci.

5. Utilisation d'un extrait d'Aframomum aulacocarpus dans la fabrication d'un médicament antipaludique ou antitrypanosoma.

6. Utilisation selon la revendication 5, où l'extrait inclut de l'aulacocarpine (composé III) extraite de celui-ci.

7. Utilisation selon l'une quelconque des revendications 1 à 5, où ledit extrait est obtenu des racines, de l'écorce des tiges, des feuilles, des fruits ou des graines desdites plantes.

8. Utilisation d'un extrait de Napoleonaea imperialis dans la fabrication d'un médicament antileishmania ou antitrypanosoma.

9. Utilisation selon la revendication 8, où ledit extrait est obtenu de graines pulvérisées de Napoleonaea imperialis.

10. Utilisation selon l'une quelconque des revendications précédentes, où ladite utilisation englobe une composition topique comprenant un extrait tel que défini dans l'une quelconque des revendications précédentes, dans un excipient topique.

11. Utilisation selon l'une quelconque des revendications précédentes, où ladite utilisation englobe une composition orale comprenant un extrait tel que défini dans l'une quelconque des revendications précédentes, dans un excipient oral.

12. Utilisation selon l'une quelconque des revendications précédentes, où ladite utilisation englobe une composition intraveineuse comprenant un extrait tel que défini dans l'une quelconque des revendications précédentes, dans un excipient intraveineux.
